# EUROPEAN PATENT APPLICATION

(11) **EP 2 425 782 A2**
(43) Date of publication of application: **07.03.2012**
(21) Application number: 10769952.2
(22) Date of filing: 28.04.2010
(51) Int. Cl.: A61B 8/00, G06T 17/40

(54) **APPARATUS AND METHOD FOR A REAL-TIME MULTI-VIEW THREE-DIMENSIONAL ULTRASONIC IMAGE USER INTERFACE FOR ULTRASONIC DIAGNOSIS SYSTEM**

(30) Priority: 30.04.2009 KR 20090038225
(71) Applicant: Alpinion Medical Systems Co., Ltd., Hwaseong-si, Gyeonggi-do 445-380 (KR)
(72) Inventor: YOO, Jinho, Seoul 142-100 (KR)
(74) Representative: Noble, Nicholas
(86) International application number: PCT/KR2010/002692
(87) International publication number: WO 2010/126307

(57) **Abstract**

The present disclosure relates to an ultrasonic diagnosis system. More particularly, the present disclosure relates to an apparatus and method for a real-time multi-view three-dimensional ultrasonic image user interface for an ultrasonic diagnosis system which can render three-dimensional volume data of an object obtained by three-dimensional ultrasonographic imaging into three-dimensional image data from each of a plurality of virtual viewpoints, and display the object from each of the viewpoints simultaneously on a real-time basis.

## Description

### [Technical Field]

The present disclosure relates to an ultrasonic diagnosis system. More particularly, the present disclosure relates to an apparatus and method for a real-time multi-view three-dimensional ultrasonic image user interface for an ultrasonic diagnosis system which can render three-dimensional object volume data obtained by three-dimensional ultrasonographic imaging into three-dimensional image data at each of a plurality of virtual viewpoints, and display the object at each of the viewpoints simultaneously on a real-time basis.

### [Background Art]

The statements in this section merely provide background information related to the present disclosure and may not constitute the prior art.

As medical electronics advance, ultrasound diagnostic systems have become widely used with applied ultrasonic measurement technology which allows observations of the interior of body of an object such as human, animal, or others. The usual operation of such ultrasound diagnostic systems is to send ultrasonic waves to the object and detect the rebound ultrasonic waves in relation to the transmitted ultrasonic signal. Typically, the image of the object is two-dimensional.

However, a recently developed 3D ultrasonic diagnosis system in the market has ultrasonic waves generated at a probe and a number of ultrasonic signals transmitted through a plurality of scan lines to an object, and acquires 3-dimensional or 3D volume data from the received reflections of the ultrasonic signals. The acquired 3D volume data from such 3D ultrasonic diagnosis system is one that is obtained by having the position of the probe for emitting the ultrasonic waves as a point of reference. The obtained 3D volume data undergoes an image processing to be displayed in a single 3D image. Such 3D ultrasonic diagnosis system provides real-time clinical data of objects such as spatial information and anatomical shapes which are unprecedented in typical 2D images.

As described above, since the conventional 3D ultrasonic diagnosis system shows the 3D images by just a unidirectional view point, it has been inconvenient for a diagnostician seeking accurate diagnosis from multidirectional 3D images to painstakingly change the face of the probe before being allowed to inspect the 3D image while holding a photographic memory of the previous 3D images.

### [Disclosure]

### [Technical Problem]

Therefore, the present disclosure is made to provide an apparatus and method for a real-time multi-view 3D ultrasonic image user interface for an ultrasonic diagnosis system which renders an object observable from multi-view points in real time and simultaneously by using 3D volume data obtained by 3D ultrasonography.

### [Technical Solution]

To implement the present disclosure, a real-time multi-view 3D ultrasonic image user interface apparatus performs a rendering of 3D volume data of an object obtained by a 3D ultrasonic diagnostic apparatus into 3D ultrasonic image data from one or more respective virtual viewpoints inputted by a user, and simultaneously displaying the 3D ultrasonic image data.

The real-time multi-view 3D ultrasonic image user interface apparatus further receives data of effect for the one or more virtual viewpoints, and, in response to the virtual viewpoints with the data of effect inputted, performs a rendering of the 3D ultrasonic image data reflecting the effect, and then displays the same 3D ultrasonic image data simultaneously with other 3D ultrasonic image data of different virtual viewpoints.

The real-time multi-view 3D ultrasonic image user interface apparatus includes: an input unit for receiving and outputting user inputs of the number of virtual viewpoints, locations of the virtual viewpoints, and split screen locations of split screens having designations of the locations of the virtual viewpoints; a 3D volume data storage unit for storing 3D volume data of an object obtained by an ultrasonic generation from the ultrasonic diagnostic apparatus; a rendering unit for receiving the user inputs of the number of virtual viewpoints and the locations of the virtual viewpoints and activating viewpoint processors corresponding to the number of virtual viewpoints and then providing the locations of the virtual viewpoints respectively to the viewpoint processors after being activated to process rendering and then simultaneous outputting of the 3D volume data into 3D ultrasonic image data from corresponding virtual viewpoints; a display composition unit for receiving the inputs of the number of the virtual viewpoints to compose a display having the split screens corresponding to the number of the virtual viewpoints, and in response to one or more inputs of 3D ultrasonic data up to a number corresponding to the virtual viewpoints and the split screen locations of the 3D ultrasonic data, for outputting display data containing the 3D ultrasonic image data in a corresponding arrangement to the split screens; a controller for receiving inputs of the number of the virtual viewpoints and the locations of the virtual viewpoints from the input unit to provide the inputs to the rendering unit and providing the display composition unit with the split screens locations inputted from the input unit to output the display data containing the 3D ultrasonic image data of the object from multiple viewpoints simultaneously; and a display unit for receiving and displaying the display data.

The controller performs displaying 3D images of the object on the display unit in a multi-viewpoint mode followed by receiving one or more virtual viewpoints inputted from a user, and controlling the rendering unit to activate the viewpoint processors for rendering the 3D volume data to generate the 3D ultrasonic image data from the virtual viewpoints.

In an aspect, the designations of the locations of the virtual viewpoints are performed by first using the input unit to rotate the object displayed on the display unit.

In another aspect, the designations of the locations of the virtual viewpoints are performed using the input unit to three-dimensionally rotate a cursor for designating the locations of the virtual viewpoints about the object displayed on the display unit.

The controller, after controlling the display composition unit to make the split screens by the number of the virtual viewpoints followed by displaying the object on a single split screen of the split screens, receives the virtual viewpoints inputted for the single split screen, and controls the rendering unit to have the single split screen display the 3D ultrasonic image data obtained by activating the viewpoint processors for generating the 3D ultrasonic image data from the virtual viewpoints.

The controller, after displaying the object on the single split screen, in response to a selection of a following second split screen, has the second split screen display an object from the virtual viewpoints shared by the object displayed in a previous split screen, and then receives the designations of the locations of the virtual viewpoints having the former object on the second spilt screen as a reference point.

The controller, when using the viewpoint processors in rendering the 3D volume data to display the 3D ultrasonic image data, further receives a rendering parameter or data of effect through the input unit to generate the 3D ultrasonic image data reflecting the rendering parameter or the data of effect.

In yet another aspect, the virtual viewpoints are determined by manipulating a mouse pointer.

In yet another aspect, the virtual viewpoints are determined by manipulating a key input device.

In yet another aspect, operating a particular key of the key input device turns the split screens from an arbitrary split screen to a next split screen.

To implement the present disclosure, a real-time multi-view 3D ultrasonic image user interface method renders 3D volume data of an object obtained in a multi-view mode by a 3D ultrasonic diagnostic apparatus into 3D ultrasonic image data from one or more respective virtual viewpoints inputted by a user, and then simultaneously displays the same 3D ultrasonic image data.

The real-time multi-view 3D ultrasonic image user interface method includes: setting a multi-view mode; splitting a screen upon receiving the number of split screens; initially displaying a first object of a first 3D ultrasonic image data after being generated by rendering 3D volume data, on an arbitrary first split screen after being selected from the split screens; subsequent to displaying the first object and through an input unit, designating locations of virtual viewpoints of the first object after being displayed; and subsequent to rendering the 3D volume data with respect to designated virtual viewpoints to regenerate the first 3D ultrasonic image data, displaying an object of the first 3D ultrasonic image data after being regenerated.

The real-time multi-view 3D ultrasonic image user interface method further includes simultaneously displaying objects corresponding to the 3D ultrasonic image data on corresponding split screens subsequent to displaying the first object on the arbitrary first split screen, receiving the virtual viewpoints inputted respectively with respect to the remainder of the split screens, and then rendering the 3D volume data for received virtual viewpoints for generating the 3D ultrasonic image data.

The real-time multi-view 3D ultrasonic image user interface method further includes setting parameters by further receiving rendering parameters or data of effect through the input unit at the designations of the locations of the virtual viewpoints to carry out rendering with the rendering parameter or the data of effect reflected and thereby display the objects reflecting the rendering parameter or the data of effect.

### [Advantageous Effects]

As mentioned above, the present disclosure obviates the need to reposition the probe multiple times in ultrasonographic imaging, which helps diagnosticians to save their operation time offering a substantial convenience.

In addition, the present disclosure enables simultaneous viewing of 3D images with multiple view angles from different viewpoints and eventually allows diagnosticians to give more accurate diagnoses than before.

### [Description of Drawings]

FIG. 1 is a diagram illustrating a configuration of an ultrasonic diagnosis system including a real-time 3D ultrasonic image user interface apparatus according to the present disclosure;

FIG. 2 is a flow diagram illustrating a method for providing a real-time 3D ultrasonic image user interface according to first aspect;

FIG. 3 is a flow diagram illustrating a method for providing a real-time 3D ultrasonic image user interface according to second aspect;

FIG. 4 is a diagram illustrating a case of 3D image of an object for one viewpoint on a split screen according to the present disclosure;

FIG. 5 is a diagram illustrating another case of 3D image of an object for two viewpoints on split screens according to the present disclosure;

FIG. 6 is a diagram illustrating yet another case of 3D image of an object for two viewpoints having different rendering parameters on split screens according to the present disclosure; and

FIG. 7 is a diagram illustrating yet another case of 3D image of an object for four viewpoints having different rendering parameters on split screens according to the present disclosure.

### [Mode for Invention]

Hereinafter, a configuration of real-time 3D ultrasonic image user interface apparatus according to the present disclosure will be described with reference to the accompanying drawings.

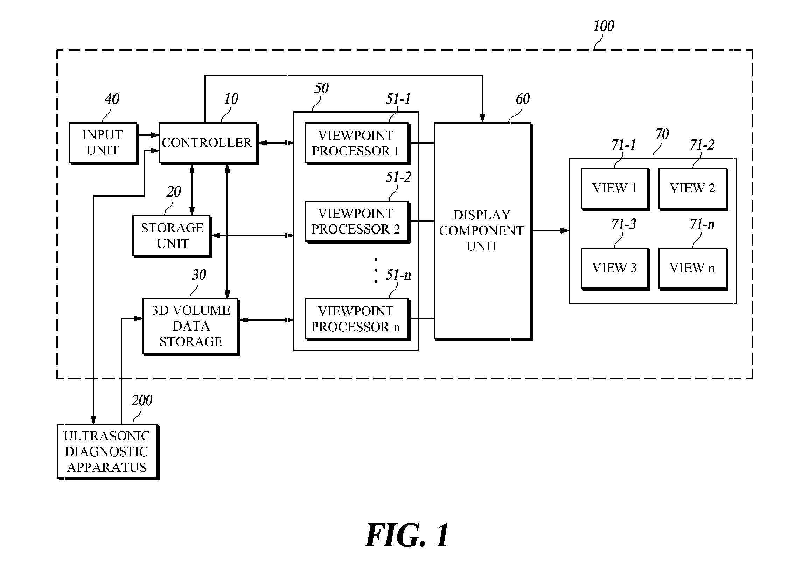

FIG. 1 schematically shows a configuration of an ultrasonic diagnosis system including a real-time 3D ultrasonic image user interface apparatus according to the present disclosure.

The ultrasonic diagnosis system comprises an ultrasonic diagnostic apparatus 200 and a real-time multi-view 3D ultrasonic image user interface apparatus (or simply called 'user interface apparatus') 100.

Ultrasonic diagnostic apparatus 200 has a probe and a number of scan lines through which it transmits ultrasonic waves, and receives reflections of the ultrasonic waves to generate and output real-time 3D volume data to user interface apparatus 100. The 3D volume data contains type of probe, working distance, depth, width, open angle, dimension of a slice in landscape and portrait, number of samples, total volume data size, etc. It should be noted that the method of composition and acquisition of the above 3D volume data is obvious in this field of art and its detailed description is not repeated herein.

User interface apparatus 100 comprises a controller 10, a storage unit 20, a 3D volume data storage unit 30, an input unit 40, a rendering unit 50, display component unit 60, and a display unit 70 so that it performs rendering with respect to viewpoints designated by a user or diagnostician to generate pieces of 3D ultrasonic image data and simultaneously display them.

Controller 10 controls the general operation of user interface apparatus 100 according to the present disclosure. Especially, controller 10 takes control of rendering unit 50, and manages overall parameters for the real-time multi-view 3D ultrasonic image user interface. Such parameters may be the number of virtual views or viewpoints, respective locations of the virtual viewpoints, rendering scheme (parameter), effect parameter, the storage location of the volume data, and others. Examples of the effect parameters may be a volume transparency or image transparency, volume threshold for removing noises, volume color, volume contrast, etc. whereas examples of the rendering parameters may be a surface rendering, X-ray, inversion among others. The obviously known techniques among individuals skilled in this art will not be repeated herein. The locations of the virtual viewpoints mean information on the direction of viewing the object.

Storage unit 20 comprises a control program area for storing a control program for controlling the operation of user interface apparatus 100, a temporary area for a temporary storage of data generated in an execution of the control program, and a data area for storing user data generated by the parameters and the user.

3D volume data storage unit 30 receives and stores the 3D volume data inputted in real time from ultrasonic diagnostic apparatus 200 and outputs the 3D volume data under the control of controller 10 to rendering unit 50.

Input unit 40 may be a keyboard having a plurality of keys for setting the parameters such as the number of virtual viewpoints, locations of the virtual viewpoints, and rendering scheme to output key signals in response to depressions of keys, a computer mouse for controlling the locations of a cursor and outputting a selected signal at an arbitrary cursor location, and/or a touchpad installed in a size corresponding to display unit 70 to output coordinate data with respect to local depressions on display unit 70.

Having a number of viewpoint processors 51 for rendering the 3D volume data to output 3D ultrasonic image data, rendering unit 50 activates viewpoint processors 51 of the number corresponding to inputted viewpoints under the control of controller 10, and reads in the 3D volume data stored in real time in 3D volume data storage unit 30 to transform the data into 3D ultrasonic image data corresponding to the viewpoint locations inputted respectively through the activated viewpoint processors 51 and output the 3D ultrasonic image data. The respective viewpoint processors 51 receive the 3D volume data inputted from 3D volume data storage unit 30 and receive the viewpoint locations from controller 10 to transform and output the 3D volume data as the 3D ultrasonic image data. In addition, rendering unit 50 receives data (parameter) of effect and/or rendering parameter inputted from controller 10, and performs a rendering of the 3D ultrasonic image data to output reflecting the parameter of effect and/or rendering parameter inputted.

Display component unit 60 receives the number of virtual viewpoints or the number of splits of a screen, the respective positions of the spilt screens, and information on the viewpoint processors corresponding to the respective split screen positions inputted from controller 10, and generates display data comprising the number of split screens, split screen positions, and pictures by the 3D image data to be displayed on the respective spilt screens, and eventually outputs the 3D image data to display unit 70.

Display unit 70 receives and displays the display data. As shown in FIG. 1, display unit 70 may provide the split screens for viewpoint 1 to viewpoint n (n is integer, n=1, 2, 3...) in a multi-viewpoint mode, and displays 3D images corresponding respectively to the split screens.

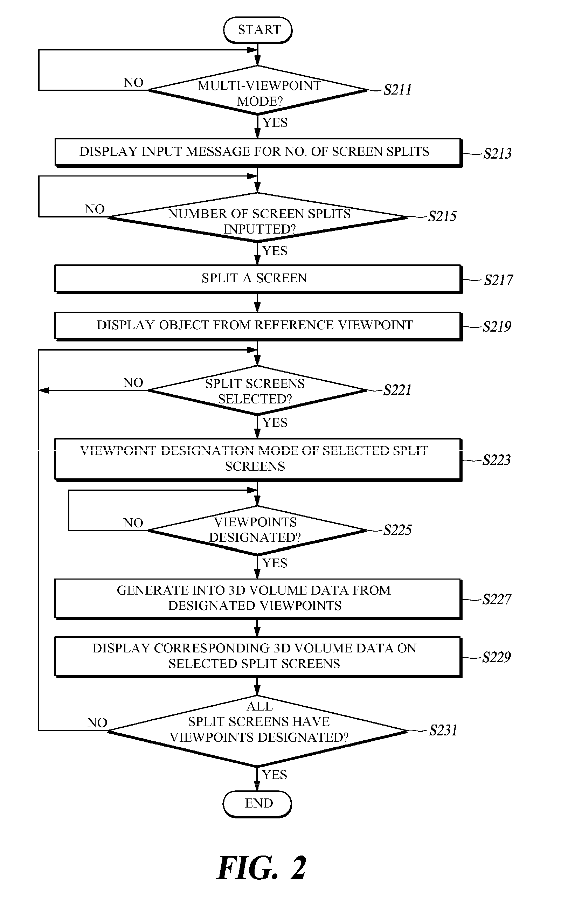

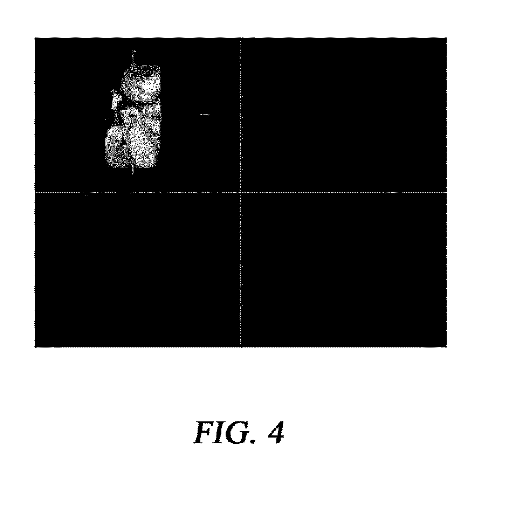

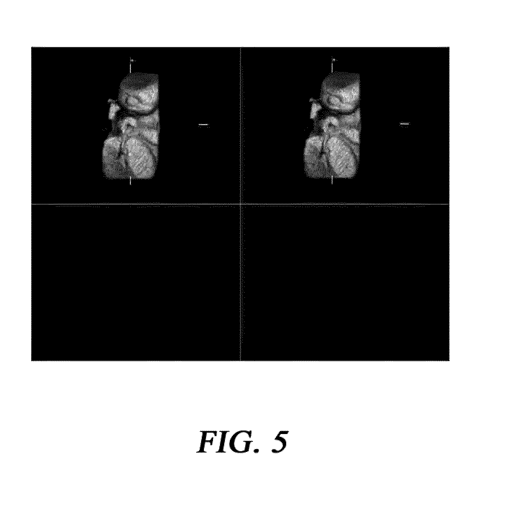

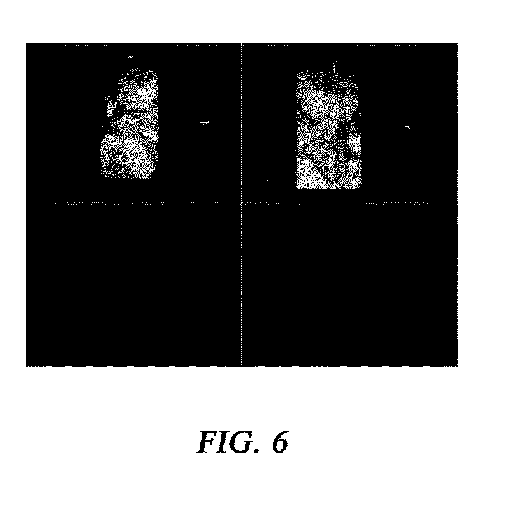

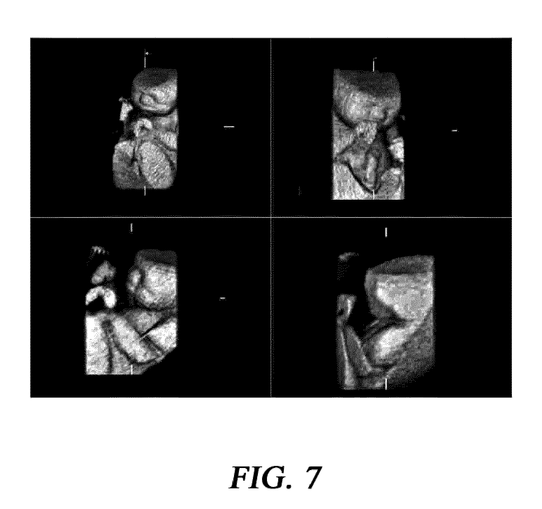

FIG. 2 is a flow diagram illustrating a method for providing a real-time multi-view 3D ultrasonic image user interface of the real-time multi-view 3D ultrasonic image user interface apparatus according to first aspect, and FIGs 4 to 7 are illustrations of the multi-view 3D images, wherein FIG. 4 is a diagram illustrating a case of 3D image of an object from one viewpoint on a split screen according to the present disclosure, FIG. 5 is a diagram illustrating another case of 3D image of the object from two viewpoints on the split screens according to the present disclosure, FIG. 6 is a diagram illustrating yet another case of 3D image of the object from two viewpoints having different rendering parameters on the split screens according to the present disclosure; and FIG. 7 is a diagram illustrating yet another case of 3D image of the object from four viewpoints having different rendering parameters on the split screens according to the present disclosure.

In the following, referring to FIGs. 1 and 2 and FIGs. 4 to 7, controller 10 first checks if the multi-viewpoint mode is set up through input unit 40 in a step S211.

If the multi-viewpoint mode is set up, controller 10 display an input message for the number of screen splits which requests for an input of the number of screen splits (or the number of virtual viewpoints) (S213).

Upon displaying the input message for the number of screen splits, controller 10 checks whether the number of screen splits are inputted (S215), and if yes, controls display component unit 60 to split a screen of display unit 70 and display the split screens (S217). Besides, controller 10 may also let a configuration or other steps for providing a checked grid display of sub-display windows corresponding to the number of screen splits.

Upon splitting the screen, controller 10 controls display component unit 60 to activate viewpoint processors 51 of the number corresponding to the split screens of the inputted number. Then, viewpoint processors 51 has its viewpoint processor 1 (51-1) corresponding to a viewpoint 1 split screen (71-1) transform the 3D volume data in the process of real-time storage from 3D volume data storage unit 30 into the 3D ultrasonic image data and then display the latter image data through display component unit 60 on the viewpoint 1 split screen (71-1), as illustrated in FIG. 4 (S219). At this time, however, all of the activated viewpoint processors 51 may be allowed to read in the 3D volume data stored in 3D volume data storage unit 30 for transforming them into the 3D ultrasonic image data and thereafter have the latter image data displayed simultaneously on the viewpoint 1 split screen (71-1) through the viewpoint n split screen (71-n), as shown in FIG.7.

When the object is on the split screens, controller 10 checks if split screens are selected (S221), and if yes, in response to a selection of the split screens, enters a viewpoint designation or selection mode for making preparation for operating viewpoint processors 51 corresponding to the selected split screens (S223).

In the viewpoint designation mode, controller 10 checks if viewpoints are designated (S225), and if yes, outputs the viewpoint locations to viewpoint processors 51 which then render the 3D volume data stored in 3D volume data storage unit 30 into the 3D ultrasonic image data for the corresponding viewpoints (S227), and subsequently display the latter image data through display component unit 60 on the corresponding viewpoint split screens 71 (S229).

As described, controller 10 first selects the viewpoint split screens 71 at the respective viewpoints and inputs the viewpoint locations to display the 3D object images corresponding to the inputted viewpoint locations. However, controller 10 may also control to check whether all the split screens have the viewpoint locations selected (S231), so as to ensure that the 3D object images for the corresponding viewpoints are displayed on the entire established viewpoint split screens 71.

In addition, subsequent to displaying the 3D ultrasonic images of the object for the inputted viewpoints on the first viewpoint split screen (71-1), if the second viewpoint split screen (71-2) is clicked, it turns to display the 3D ultrasonic image data which was initially displayed on the first viewpoint split screen (71-1).

Although not described in the above process, controller 10 relays the data of effect and/or rendering parameter inputted for the 3D images on the split screens selected through input unit 40 to viewpoint processors 51 belonged to rendering unit 50 and corresponding to the split screens selected. Then, viewpoint processors 51 generate the 3D ultrasonic image data reflecting the data of effect and/or rendering parameter (S227), and subsequently display the latter image data through display component unit 60 on display unit 70 at the corresponding viewpoint split screens 71 (S229). The data of effect and rendering parameter may be inputted by the user before or after inputting the number of the screen splits or when selecting the split screens or when inputting commands for setting the effect and/or rendering parameter through input unit 40.

The rendering parameter may be a view angle and size parameters for example, and viewpoint processors 51 of rendering unit 50 display the object in varying view angles and sizes reflecting the view angle and size parameters.

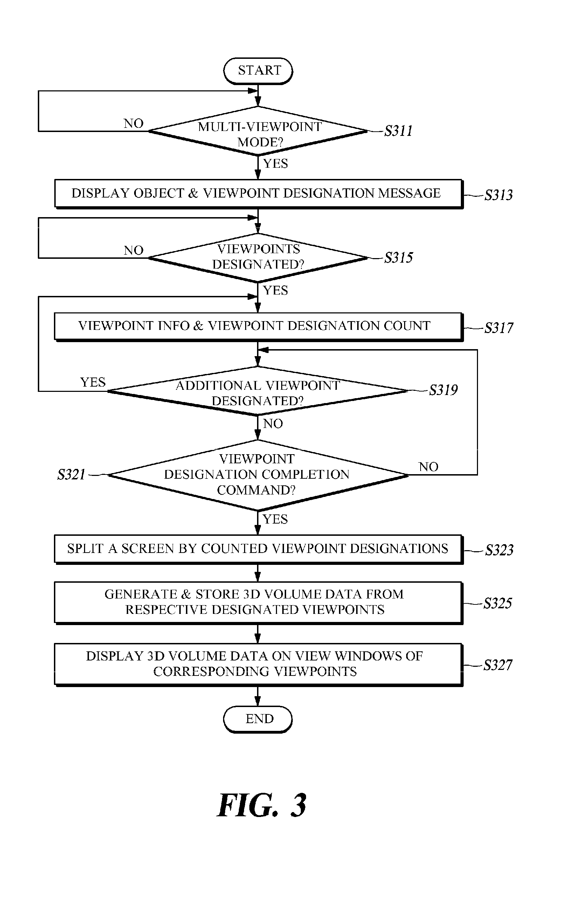

FIG. 3 is a flow diagram illustrating a method for providing a real-time 3D ultrasonic image user interface of the real-time multi-view 3D ultrasonic image user interface apparatus according to a second aspect. Following description will be presented referring to FiGs. 1, 3, 4 to 7.

At the outset, controller 10 checks if a multi-viewpoint mode is designated through input unit 40 (S311).

Upon selecting the multi-viewpoint mode, instead of splitting the screen as in FIG. 2, controller 10 first displays the object on display unit 70. Then, controller 10 displays a viewpoint designation message for requesting designating a viewpoint so that it overlaps an area or the object on display 70 (S313). The viewpoint designation message may be held from being outputted or become selective in an option.

After displaying the viewpoint designation message, controller 10 checks whether viewpoints are designated by input unit 40 (S315). Designations of these viewpoints may be carried out using the computer mouse by turning the object in pitches and rolls wherein the displayed object in such revolution may allow for a secondary designation of viewpoints. To relieve the corresponding viewpoint processors 51 from a possible large load of having to process a large amount data fast, the object may remain still in the display as the cursor or icon for designating the viewpoints is moved about the object in horizontal, vertical, and diagonal directions to input the viewpoint locations.

When the viewpoints are designated, controller 10 stores information on the designated viewpoint, i.e. the object viewing direction and counts the number of the viewpoint designations (S317).

Subsequent to counting the number of the viewpoint designations, controller 10 checks if viewpoints are additionally designated (S319) and if a viewpoint designation completion command is inputted (S321), and if the viewpoints are additionally designated, the process returns to the step (S317) for storing the viewpoint information and counting the number of the viewpoint designations which is incremental until completing the viewpoint designation at the completion command to eventually control display component unit 60 for splitting the screen corresponding to the counted viewpoint designations (S323).

Upon splitting the screen, controller 10 outputs the number of the viewpoint designations to rendering unit 50 to activate viewpoint processors 51 corresponding to the same number of the viewpoint designations. Then, viewpoint processors 51 of rendering unit 50 read in the 3D volume data in the process of real-time storage in 3D volume data storage unit 30 to perform rendering into 3D image data from the respective designated viewpoints (S325) and then output it to display component unit 60. At this time, display component unit 60 under the control of controller 10 displays the 3D image data on the corresponding split screens (S327). Controller 10 is mapping the activated viewpoint processors 51 and the split screens, and displays the 3D image data on the corresponding split screens based on the mapping information.

The second aspect as above may also select effects through an input method such as when designating the viewpoints or inputting an extra effect setting command from input unit 40, when controller 10 provides the data of effect for the selected effect to rendering unit 50 so as to output 3D image data reflecting the particular effect. Further, not only the data of effect, the 3D image data may be outputted reflecting a fresh input of rendering parameters such as 3D image view angles respectively corresponding to the viewpoints, or it may be outputted after a rendering process to meet stored rendering parameters from a previous input.

On the other hand, it would be clear to those skilled in the art that the specified types of aspects do not restrict the present disclosure which suggests various improvements, modifications, replacements or additions in the course of implementing the disclosure. Such implementations of the disclosure based on the improvements, modifications, replacements or additions as interpreted within the scope of the attached claims are technical ideas belonged to the present disclosure.

### CROSS-REFERENCE TO RELATED APPLICATION

If applicable, this application claims priority under 35 U.S.C §119(a) on Patent Application No. 10-2009-0038225 filed in Korea on April 30, 2009, the entire content of which is hereby incorporated by reference. In addition, this non-provisional application claims priority in countries, other than the U.S., with the same reason based on the Korean Patent Application, the entire content of which is hereby incorporated by reference.

## Claims

1. A real-time multi-view 3D ultrasonic image user interface apparatus for rendering 3D volume data of an object obtained by a 3D ultrasonic diagnostic apparatus into 3D ultrasonic image data from one or more respective virtual viewpoints inputted by a user, and simultaneously displaying the 3D ultrasonic image data.

2. The real-time multi-view 3D ultrasonic image user interface apparatus of claim 1 which further receives data of effect of the one or more virtual viewpoints, and, in response to the virtual viewpoints at which the data of effect is inputted, performs a rendering of the 3D ultrasonic image data reflecting the data of effect, and then displays the same 3D ultrasonic image data simultaneously with other 3D ultrasonic image data of different virtual viewpoints.

3. The real-time multi-view 3D ultrasonic image user interface apparatus of claim 1, comprising:
an input unit for receiving and outputting user inputs of the number of virtual viewpoints, locations of the virtual viewpoints, and split screen locations of split screens having designations of the locations of the virtual viewpoints;
a 3D volume data storage unit for storing 3D volume data of an object obtained by an ultrasonic generation from the ultrasonic diagnostic apparatus;
a rendering unit for receiving the user inputs of the number of virtual viewpoints and the locations of the virtual viewpoints and activating viewpoint processors corresponding to the number of virtual viewpoints and then providing the locations of the virtual viewpoints respectively to the viewpoint processors after being activated to process rendering and then simultaneous outputting of the 3D volume data into 3D ultrasonic image data from corresponding virtual viewpoints;
a display composition unit for receiving the inputs of the number of the virtual viewpoints to compose a display having the split screens corresponding to the number of the virtual viewpoints, and in response to one or more inputs of 3D ultrasonic data up to a number corresponding to the virtual viewpoints and the split screen locations of the 3D ultrasonic data, for outputting display data containing the 3D ultrasonic image data in a corresponding arrangement to the split screens;
a controller for receiving inputs of the number of the virtual viewpoints and the locations of the virtual viewpoints from the input unit to provide the inputs to the rendering unit and providing the display composition unit with the split screens locations inputted from the input unit to output the display data containing the 3D ultrasonic image data of the object from multiple viewpoints simultaneously; and
a display unit for receiving and displaying the display data.

4. The real-time multi-view 3D ultrasonic image user interface apparatus of claim 3, wherein the controller performs displaying 3D images of the object on the display unit in a multi-viewpoint mode followed by receiving one or more virtual viewpoints inputted from a user, and controlling the rendering unit to activate the viewpoint processors for rendering the 3D volume data to generate the 3D ultrasonic image data from the virtual viewpoints.

5. The real-time multi-view 3D ultrasonic image user interface apparatus of claim 4, wherein designations of the locations of the virtual viewpoints are performed by first using the input unit to rotate the object displayed on the display unit.

6. The real-time multi-view 3D ultrasonic image user interface apparatus of claim 4, wherein the designations of the locations of the virtual viewpoints are performed using the input unit to three-dimensionally rotate a cursor for designating the locations of the virtual viewpoints about the object displayed on the display unit.

7. The real-time multi-view 3D ultrasonic image user interface apparatus of claim 3, wherein the controller, after controlling the display composition unit to make the split screens by the number of the virtual viewpoints followed by displaying the object on a single split screen of the split screens, receives the virtual viewpoints inputted for the single split screen, and controls the rendering unit to have the single split screen display the 3D ultrasonic image data obtained by activating the viewpoint processors for generating the 3D ultrasonic image data from the virtual viewpoints.

8. The real-time multi-view 3D ultrasonic image user interface apparatus of claim 7, wherein the controller, after displaying the object on the single split screen, in response to a selection of a following second split screen, has the second split screen display an object from the virtual viewpoints shared by the object displayed in a previous split screen, and then receives the designations of the locations of the virtual viewpoints having the former object on the second spilt screen as a reference point.

9. The real-time multi-view 3D ultrasonic image user interface apparatus of claim 7, wherein the controller, when using the viewpoint processors in rendering the 3D volume data to display the 3D ultrasonic image data, further receives a rendering parameter or data of effect through the input unit to generate the 3D ultrasonic image data reflecting the rendering parameter or the data of effect.

10. The real-time multi-view 3D ultrasonic image user interface apparatus of claim 1 or claim 7, wherein the virtual viewpoints are determined by manipulating a mouse pointer.

11. The real-time multi-view 3D ultrasonic image user interface apparatus of claim 8, wherein the virtual viewpoints are determined by manipulating a key input device.

12. The real-time multi-view 3D ultrasonic image user interface apparatus of claim 8, wherein operating a particular key of the key input device turns the split screens from an arbitrary split screen to a next split screen.

13. A real-time multi-view 3D ultrasonic image user interface method for rendering 3D volume data of an object obtained in a multi-view mode by a 3D ultrasonic diagnostic apparatus into 3D ultrasonic image data from one or more respective virtual viewpoints inputted by a user, and then simultaneously displays the same 3D ultrasonic image data.

14. The real-time multi-view 3D ultrasonic image user interface method of claim 13, comprising:
setting a multi-view mode;
splitting a screen upon receiving the number of split screens;
initially displaying a first object of a first 3D ultrasonic image data after being generated by rendering 3D volume data, on an arbitrary first split screen after being selected from the split screens;
subsequent to displaying the first object and through an input unit, designating locations of virtual viewpoints of the first object after being displayed; and
subsequent to the rendering of the 3D volume data with respect to designated virtual viewpoints to regenerate the first 3D ultrasonic image data, displaying an object of the first 3D ultrasonic image data after being regenerated.

15. The real-time multi-view 3D ultrasonic image user interface method of claim 14, further comprising:
simultaneously displaying objects corresponding to the 3D ultrasonic image data on corresponding split screens subsequent to displaying the first object on the arbitrary first split screen, receiving the virtual viewpoints inputted respectively with respect to the remainder of the split screens, and then rendering the 3D volume data for received virtual viewpoints for generating the 3D ultrasonic image data.

16. The real-time multi-view 3D ultrasonic image user interface method of claim 14 or claim 15, further comprising:
setting parameters by further receiving rendering parameters or data of effect through the input unit at the designations of the locations of the virtual viewpoints to carry out a rendering with the rendering parameter or the data of effect reflected and thereby display the objects reflecting the rendering parameter or the data of effect.
